# EUROPEAN PATENT SPECIFICATION

(11) **EP 0 835 637 B1**
(45) Date of publication and mention of the grant of the patent: **02.07.2003**
(21) Application number: 97106748.3
(22) Date of filing: 23.04.1997
(51) Int. Cl.: A61B 10/00

(54) **Treatment accessories for an endoscope**
Behandlungszubehör für ein Endoskop
Accessoire de traitement pour endoscope

(30) Priority: 08.10.1996 JP 26718696; 13.01.1997 JP 344097; 05.02.1997 JP 2226897
(43) Date of publication of application: 15.04.1998
(73) Proprietor: Pentax Corporation, Tokyo (JP)
(72) Inventor: Ouchi, Teruo, c/o Asahi Kogaku Kogyo K.K., Itabashi-ku, Tokyo (JP)
(74) Representative: Schaumburg, Thoenes, Thurn Patentanwälte

(56) References cited:
- WO-A-96/09009
- DE-A- 2 926 339

## Description

### Background of the Invention

The present invention relates to a treatment accessory to be inserted in a forceps channel of an endoscope.

A treatment accessory for an endoscope having a flexible shaft inserted in a flexible sheath is known. A variety of types of treatment accessories are formed by securing different treating devices, such as forceps or the like, to the distal end of the flexible shaft.

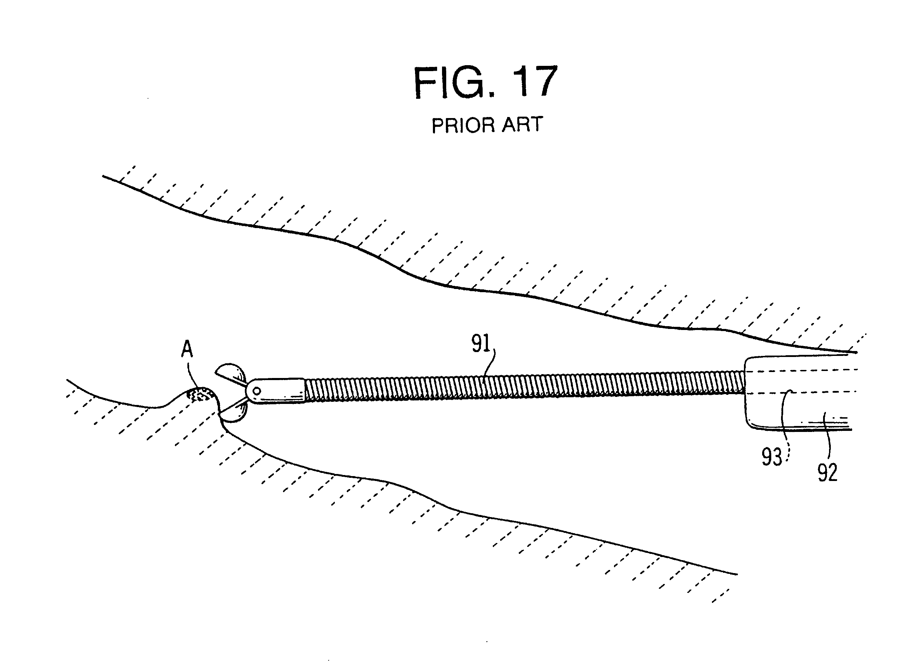

As shown in Fig. 17, when a conventional treatment accessory, in this case provided with forceps as a treating device, is used to treat an affected part A of human tissue, the distal end of the flexible shaft 91 is extended from a forceps channel 93 of an endoscope 92 straight towards the affected part A. In this example, since the affected part A is not directly in front of the treatment accessory, it is difficult to treat the affected part A accurately.

In this case, it may be necessary to press contact the distal end of the treatment accessory, i.e., attempt to hook the treatment accessory onto the tissue in the neighborhood of the affected part A, and then further extend the flexible shaft 91 from the forceps channel 93, such that the flexible shaft 91 is bent and the orientation of the distal end of the treatment accessory is changed. However, since the flexible shaft 91 of the treatment accessory is a closely wound coil having a relatively strong elasticity, the distal end of the treatment accessory may slip off the portion at which it has been press contacted or hooked. If the distal end slips, the flexible shaft 91 elastically straightens back to the position shown in Fig. 17.

In order to overcome the above problem, treatment accessories having a flexible shaft which is remotely controlled to bend in a desired direction are known. An examples of such an instrument are disclosed in Japanese Utility Model Registration Publication SHO 52-22146, Japan Utility Model Provisional Publication HEI 1-119621, and the like.

In such an instrument, however, a manipulation portion of the endoscope must include mechanisms for simultaneously performing the bending operation and the treatment of the affected portion A. Such a device is difficult for a user to operate since the positioning of the distal end of the treatment accessory in front of the affected part is difficult. Further, in such an instrument, both an operation wire for the operation of the treating device of the affected part and another operation wire for the bending of the flexible shaft are enclosed within the flexible . shaft. As such, the flexible shaft must be larger in diameter and more rigid making it more difficult to insert the flexible shaft in the forceps channel of the endoscope and then bend the flexible shaft at the appropriate position.

A further flexible surgical instrument incorporating a hollow lumen coil is disclosed in the international patent application WO 96/09009 A1. At different portions of the coils it was wound with a different pre-load tension, so that the different portions have a different stiffness.

### Summary of the Invention

It is therefore an object of the present invention to provide an improved treatment accessory for an endoscope which is easy to insert in a forceps channel and which allows easy adjustment of the position of a distal end of the treatment accessory in relation to an affected part with a simple structure.

In order to solve the above problem, according to the present invention, there is provided a treatment apparatus for use with an endoscope, the treatment apparatus being inserted in a forceps channel of the endoscope, the treatment apparatus comprising: a treatment device; an elongated elastic element connected to the treatment device at a distal end of the elastic element, the elastic element including at least two bendable portions, a first bendable portion having a predetermined flexibility, a second bendable portion having a greater flexibility and being shorter than the first bendable portion, the second bendable portion being located between the first bendable portion and the treatment device, at least a portion of the first bendable portion and the entire second bendable portion protruding from a distal end of the forceps channel when the treatment device is in use.

Since the second bendable portion allows the flexible element to be bent more easily in order to adjust the position of the treatment device such as the forceps with respect to an affected part.

In one embodiment, the first and second bendable portions comprise wound coils, and an outer surface of the coils of the second bendable portion is grounded. Accordingly, the second bendable portion is easier to be bent even if the same force is applied to the first and second bendable portions.

The second bendable portion may be formed to have more loosely wound coil than the first bendable portion. In this case, the second bendable portion could be grounded or not.

Further, the coil of the second bendable portion may be formed of thinner wire than the first bendable portion.

The first and second bendable portions comprise flexible sheaths, and a mesh material being embedded in only the first bendable portion. Accordingly, the first bendable portion could be less flexible than the second bendable portion, i.e., the second bendable portion could be easier to be bent than the first bendable portion.

In this case, the second bendable portion could be formed to have wound coils.

The first and second bendable portions described above could be formed of different materials.

In an example, the length of the second bendable portion is equal to or less than 50mm. This length could be determined arbitrarily, however, if the length is too long, the second bendable portion may not function sufficiently. Thus, it is preferable that the length of the second bendable portion may be equal to or less than 50 mm.

The treatment device could have grasping jaws, and at least one projection is provided on each of the grasping jaws. The projection helps the second bendable portion to function well. That is, the projection contacts the object to be treated and anchors the treatment device so that, as the flexible element is pushed, the second bendable portion bends appropriately, with less risk that the treatment device will slide along the object to be treated, even if the treatment device approaches the object to be treated at an oblique angle.

If the projection does not extend beyond a plane perpendicular to a distal end of the grasping jaws, when the second bendable portion bends and the treatment device rotates about the projection, the jaws can be located relatively accurately at the affected part.

The jaws contact each other at a contact portion when closed, and each of the projections may be spaced from the contact portion.

Optionally, at least one of the projections may be half-ring shaped.

Further, at least one of the projections may be cone-shaped.

It is also possible that a plurality of projections could be provided on at least one of the jaws.

### Brief Description of the Drawings

Fig. 1 is a side view of an endoscope having a forceps channel, in which a treatment accessory is inserted;
Fig. 2 is a side view of the treatment accessory according to a first example;
Fig. 3 is a sectional side view of a distal end of a treatment accessory according to the first example ;
Fig. 4 shows the distal end of the treatment accessory when the forceps cups are opened;
Fig. 5 is a sectional side view of forceps cups of the treatment accessory;
Fig. 6 is a front view of the forceps cups of Fig. 5;
Fig. 7 is a schematic view illustrating the operation of the treatment accessory shown in Fig. 3 when preparing to collect tissue;
Fig. 8 is a schematic view illustrating the operation of the treatment accessory shown in Fig. 3 while collecting tissue;
Figs. 9, 10, 11 show alternative arrangements of the treatment accessory according to the first example;
Fig. 12 is a sectional view of a distal end of a treatment accessory according to the second example having an alternative structure;
Fig. 13 is a sectional side view of a distal end of a treatment accessory according to a third example ;
Fig. 14 is a sectional view of a distal end of a treatment accessory according to a fourth example;
Fig. 15 is a sectional view of a distal end of a treatment accessory according to the fifth example;
Fig. 16 is a sectional view of a distal end of a treatment accessory according to a sixth example; and
Fig. 17 is a schematic view illustrating the use of a conventional treatment accessory;

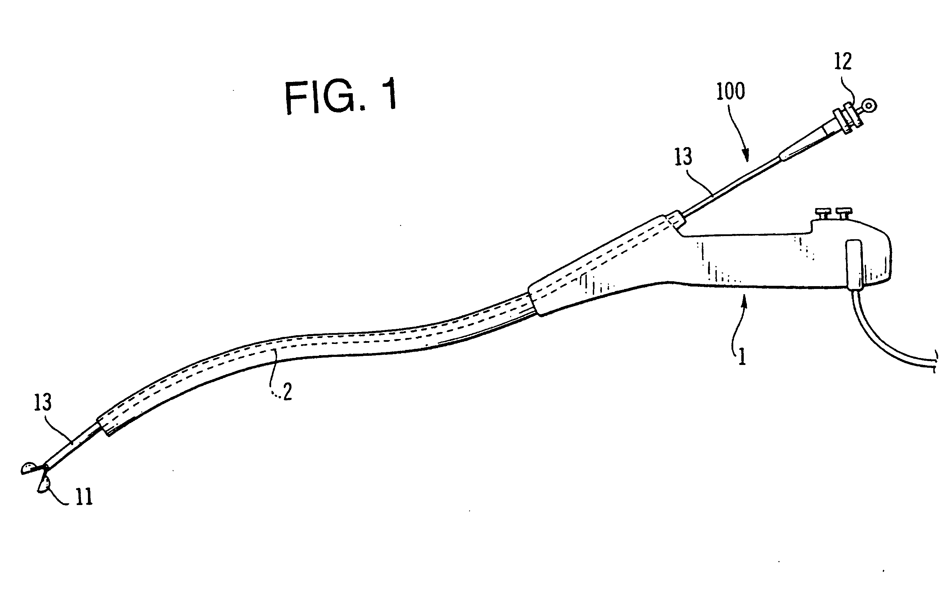

Fig. 1 shows a schematic view of an endoscope 1 and a treatment accessory 100. The treatment accessory 100 is, when in use, inserted in a forceps channel 2 formed in the endoscope 1.

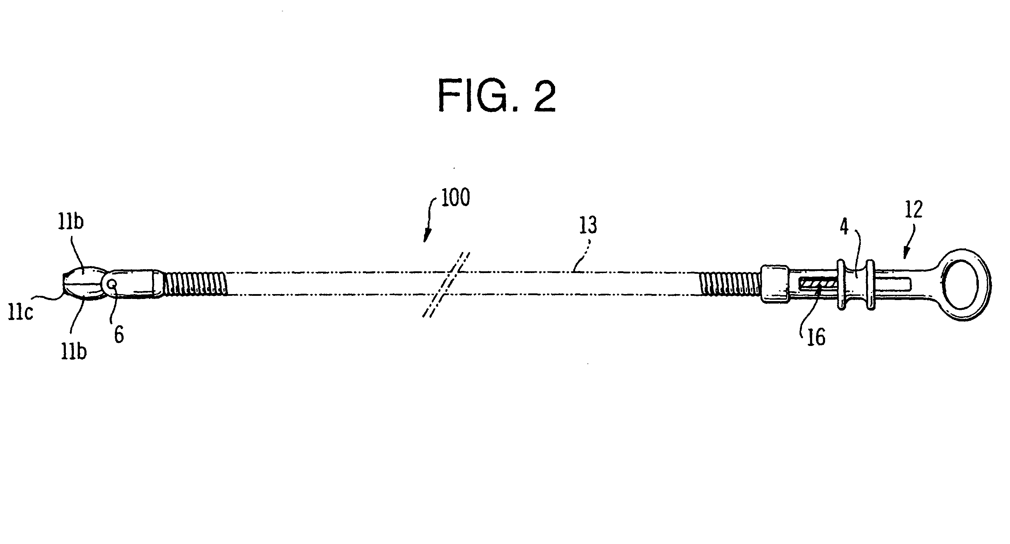

Fig. 2 shows the treatment accessory 100 according to a first example. The treatment accessory 100 includes a flexible shaft 13 made from, for example a closely wound stainless-steel coil. Note that the flexible shaft 13 is neutrally straight, and when a certain force is applied, it bends. As will be described later, the flexible shaft 13 has a portion which is easier to be bent than the other portion. Accordingly, when a certain force is applied to the shaft 13, the portion which is easier to be bent bends firstly.

A treatment device 11 is provided at the distal end of the shaft 13, and a manipulation portion 12 is provided at the proximal end of the shaft 13. In this example, the treatment device 11 is a pair of biopsy forceps, however, other treatment devices having a similar grasping, cutting function may also be provided.

The treatment is connected with a wire 16, the other end of the wire 6 being connected to a slider 4 which is to be slidably operated by an operator. By sliding the slider 4, the treatment device 11 can be operated. In the example, the biopsy forceps can be opened or closed by sliding the slider 4.

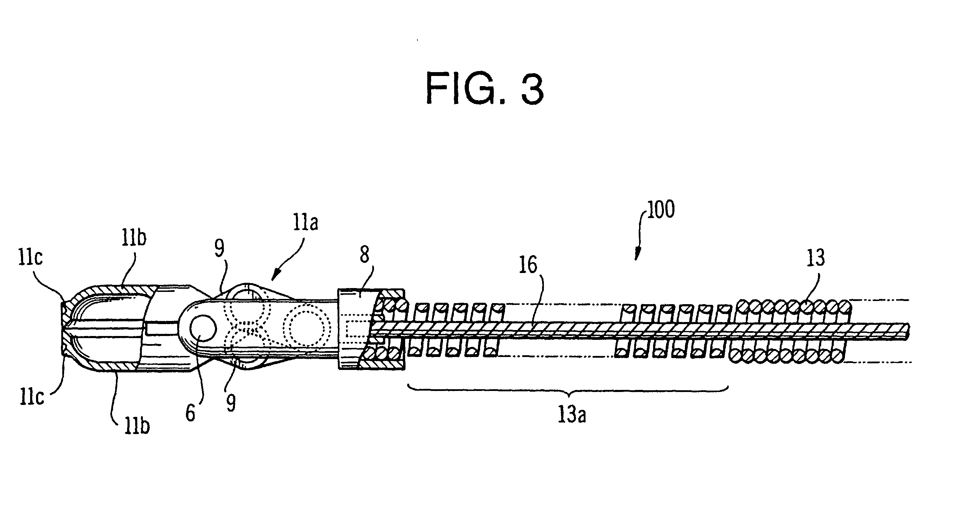

Fig. 3 shows a detailed arrangement of the treatment accessory 100. The treatment accessory 100 includes a pair of forceps cups 11b which are rotatably mounted to the link shaft 6. The forceps cups 11b and the link shaft 6 are connected with the wire 16 through the linking mechanism 11a. As described above, by operating the slider 4 provided at the manipulation portion 12 (see Fig. 2), the wire 16 moves along a central axis of the treatment accessory 100 and accordingly opens and closes the forceps cups 11b. The wire 16 is inserted in a flexible shaft 13 which is formed from a closely wound stainless steel, or the like.

The forceps cups 11b are formed, such that, when closed, the forceps cups 11b have an elliptical shape, one of the short ends of which is attached to the link shaft 6, the other of the short ends being a tip portion. The edges of the forceps cups 11b that meet when the forceps cups 11b are closed are formed as cutting blades.

The treatment device 11 includes a main body 8 that is fixedly connected at the distal end of the shaft 13. The link shaft 6 is provided at the distal end portion of the main body 8. The main body 8 is formed such that a rear portion (the right-hand side in Fig. 3) thereof is cylindrical, and a front portion (the left-hand side in Fig. 3) has parallel arms spaced to define a slit.

The forceps cups 11b are each formed to include an arm 9 which extends to the rear side (right-hand side in Fig. 3). The arms 9 form a part of the pantograph-type link mechanism 11a. Further, the distal end of the wire 16 is connected to the rear end of the link mechanism 11a. In this way, the forceps cups 11b are connected to the wire 16 by a known linking mechanism, such as the pantograph-type link mechanism 11a.

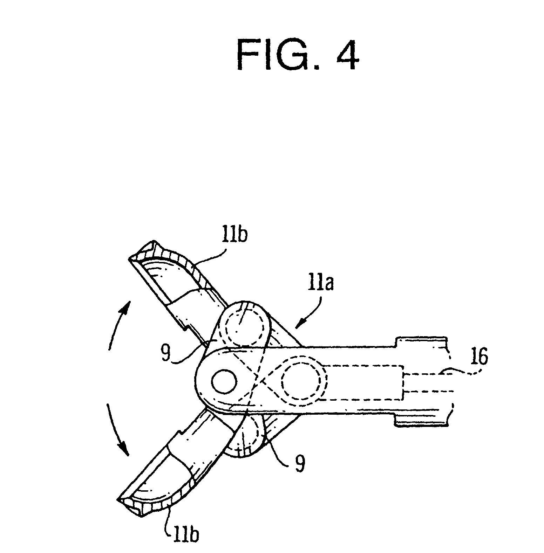

Accordingly, when the slide 4 is operated so that the wire 16 is pushed out of the flexible shaft 13, the link mechanism 11a operates to open the forceps cups 11b as shown in Fig. 4. Conversely, when the wire 16 is pulled, the forceps cups 11b are drawn closed as shown in Fig. 3. If tissue or the like is located at the forceps cups 11b during closing, the tissue is cut by the blades on the forceps cups 11b and collected inside the forceps cups 11b.

The treatment accessory 100 is further provided with an easy-to-bend (ETB) portion 13a that is formed at the distal end portion of the shaft 13. According to the first example, the outer surface of the coil that forms the shaft 13 may be ground at the easy-to-bend (ETB) portion 13a such that the coil has less sectional surface area than the other portions of the coil.

Further, the shaft 13, except for the easy-to-bend portion 13a, is preloaded such that adjacent windings in the coil contact with each other in order to increase the stiffness thereof. As a result, the shaft 13 is relatively stiff and straight while the ETB (easy-to-bend) portion 13a is more flexible and can be bent relatively easily in any direction in comparison to the other portion of the shaft 13. Note that there is no operation wire provided for controlling the bending of the ETB portion 13a. In the first example, the flexibility of the ETB portion 13a is more than twice that of the main portion of the shaft 13. That is, when applying a force perpendicular to the axis of the shaft 13, less than half the force necessary to bend the main portion of the shaft 13 is required to bend the ETB portion 13a. Of course, if this property of flexibility can be achieved using a preloaded ETB portion 13a, such an alternative structure is also acceptable.

As an example, the length of the shaft 13 may be 1 - 2 meters, and the length of the ETB portion 13a may be 5 -30 mm. Further, the preloading described above may be in the range of 100 - 150 grams.

It should be noted that, when the shaft 13 is pushed, the ETB portion 13a should be bent easily, while the other part of the shaft 13 should not be bent so much, in order to adjust the orientation of the treatment device 11. If the ETB portion is too long with respect to the portion extended from the forceps channel 2, the ETB portion may not function well. In this aspect, it is preferable that the ETB portion 13a is not so long, and may be equal to or less than 50 mm.

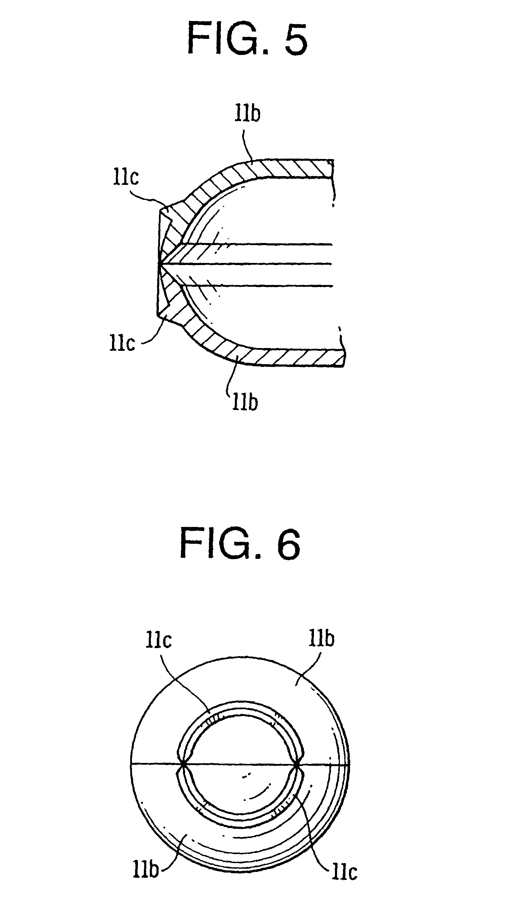

As shown in Figs. 5 and 6, a projected portion 11c is formed near the tip of each of the forceps cups 11b. The projected portion 11c is directed to a front side (left-hand side in Fig. 5). Further, as shown in Fig. 6, when the forceps cups 11b are closed, the projected portions 11c form a ring shape having a diameter that is approximately half of the maximum diameter of the forceps cups 11b when viewed from the front, as shown in Fig. 6. The tips of the projected portions 11c and the tip of the forceps cups 11b are substantially on the same plane. In other words, the tips of the projected portions 11c do not protrude beyond the tip of the forceps cups 11b.

Figs. 38 and 39 show the collection of tissue using the treatment accessory 100.

When an affected part A is identified, the distal end of the treatment accessory 300 is projected from the forceps channel 2 of the endoscope 1 and the forceps cups 11b are opened.

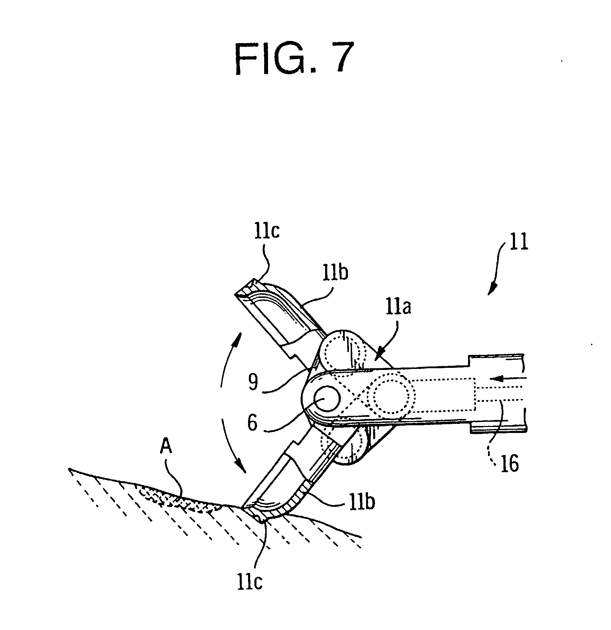

In this case, since the forceps cups 11b are directed to the affected part A from an oblique angle, first, the projected portion 11c of one of the forceps cups 11b is pushed slightly into the tissue near the affected area A as shown in Fig. 7. At this stage, since the tip of the projected portion 11c does not protrude beyond the tip of the forceps cups 11b, the tip of the forceps cups 11b is brought into contact with the tissue.

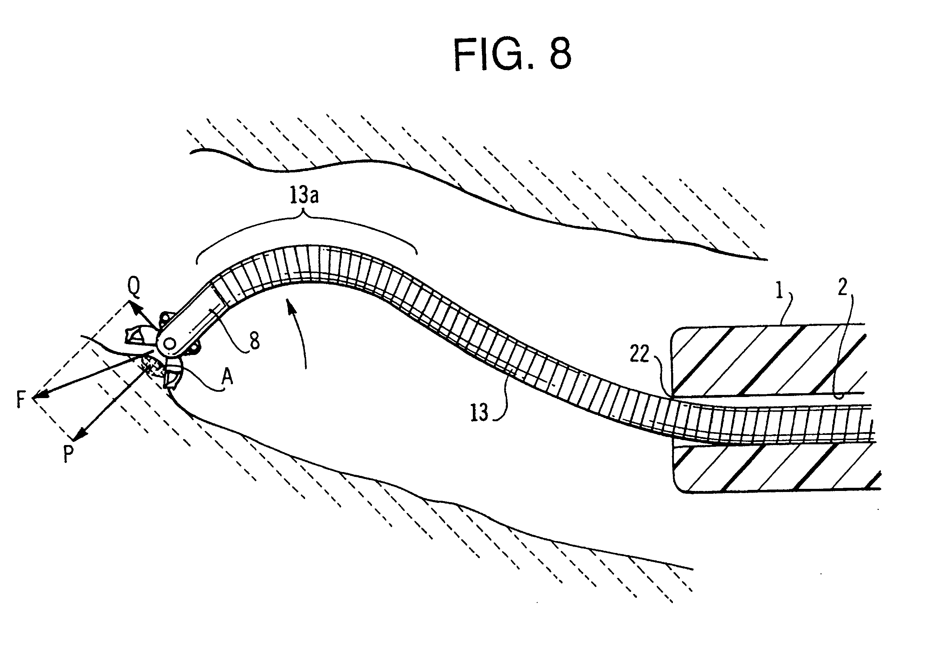

By further pressing the shaft 13 through the forceps channel 2, the ETB portion 13a bends smoothly to form a curve smoothly connecting the outlet 22 of the forceps channel 2 to the main body 8 of the forceps cups 11b while, at the same time, the attitude of the treatment accessory 100 is changed such that the treatment device 11 approaches the affected part A from above (in the view of Fig. 8). When the endoscope 1 and treatment accessory 100 are positioned as shown in Fig. 8, a force P is applied by the forceps cups 11b to the affected part A in the direction of the axis of the main body 8 and a force Q, directed perpendicular to the force P, is exerted by the ETB portion 13a due to a restoring force causing the ETB portion 13a and the shaft 13 to straighten. Thus, a resultant force F is applied to the affected part A as shown in Fig. 8. Therefore, the forceps cups 11b are strongly pressed against the affected part A. Accordingly, in this case, in which the treatment device 11 is the biopsy forceps, the tissue at the affected part A can be collected. Further, if another type of treatment device, other than the biopsy forceps, is used, the affected part A can also be treated appropriately and accurately since the attitude of the treatment device is adjusted by the ETB portion 13a regardless of the type of the treatment device used.

As another particular example, the length of the ETB portion 13a for an alimentary canal is preferably 30 mm or less. Note that, if the length of the ETB portion 13a is too long, the ETB portion 13a may not bend appropriately and further, in order to obtain an effect as described above, the length should be at least 5 mm.

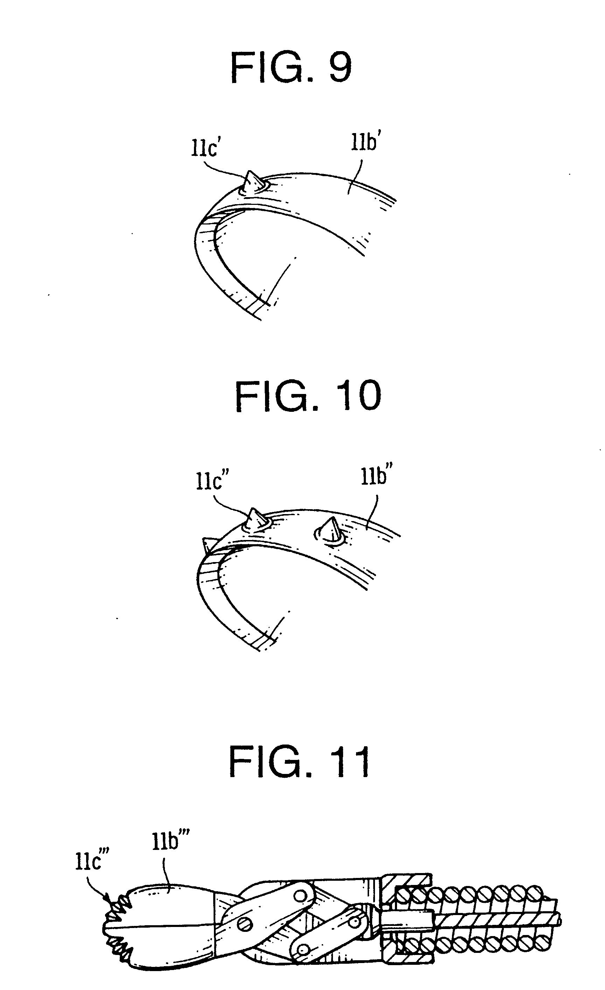

Fig. 9 shows an alternative forceps cup 11b' having a projected portion 11c'. In this alternative, instead of a continuous half-ring-shaped projected portion 11c as described above, at least one cone-shaped pointing portion 11c' is formed to serve a similar function.

Fig. 10 shows another alternative forceps cup 11b" having projected portions 11c". In this alternative, instead of a single projected portion 11c' as described above, a plurality of cone-shaped pointing portions 11c" are formed to serve a similar function.

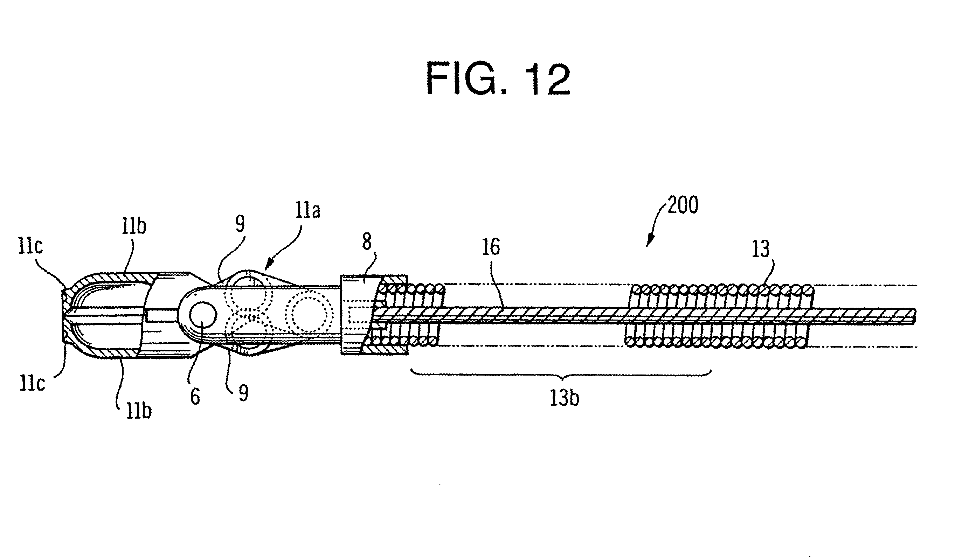

Fig. 11 shows further alternative forceps cup 11b'" having a plurality of grooves such that the forceps cup 11b'" is formed to be a corrugated surface 11c'". In this alternative, instead of a single projected portion 11c' or a plurality of projected portions 11c", a plurality of grooves are formed such that the surface of the forceps cup is provided with a plurality of projected parts and grooves, i.e., the surface is formed to have the corrugated surface 11c'". Such a surface also serves the similar function.

As with the previous example, the projected portion 11c is not limited to the examples described, and, for example, may be modified as described above or according to the nature of the affected part A. For example, if the affected part includes relatively hard material such as a tumor or the like, or if the affected part is located at a slippery area (e.g., mucous covered surface), the projected portion 11c may be modified accordingly in order to firmly fix the position of the treatment device 11 with respect to the affected part. Further, a combination of various types of projected portions may also be applicable.

In the present example, the treatment accessory is described as including forceps cups 11b, however, other types of treatment accessories can also be formed to include a projected portion 11c (or alternatives) such as described above. For example, the treatment accessory may have grasping forceps as the treatment device.

Fig. 12 shows a treatment accessory 200 according to a second example. In the treatment accessory 200, an ETB (Easy-To-Bend) portion 13b is formed using a coil that has a smaller diameter than the diameter of the main portion of the shaft 13. This can be achieved by using a wire which has a diameter that changes at a predetermined point.

As above, preferably, there should be no preloading at the ETB portion 13b. However, if the flexibility of the ETB portion 13b will be more than twice that of the main portion of the shaft 13 even after preloading, preloading at the ETB portion 13b can be performed.

In the second example, the ETB portion 13b is described as a closely wound portion, however, it is also possible to form the ETB portion 13b as a non-dense portion where the adjacent windings have spaces therebetween.

In the second example, since the shaft 13 includes the ETB portion 13b, even if the treatment accessory 200 approaches the affected part A from an oblique angle, by pressing the shaft 13 further out of the forceps channel 2, the attitude of the treatment device 11, e.g., the forceps cups 11b, can be adjusted to be appropriately positioned with respect to the affected part A.

Further, since no extra wire is required for control of the ETB portion 13a, the treatment accessory 100 is thin and can be inserted in the forceps channel 2 easily.

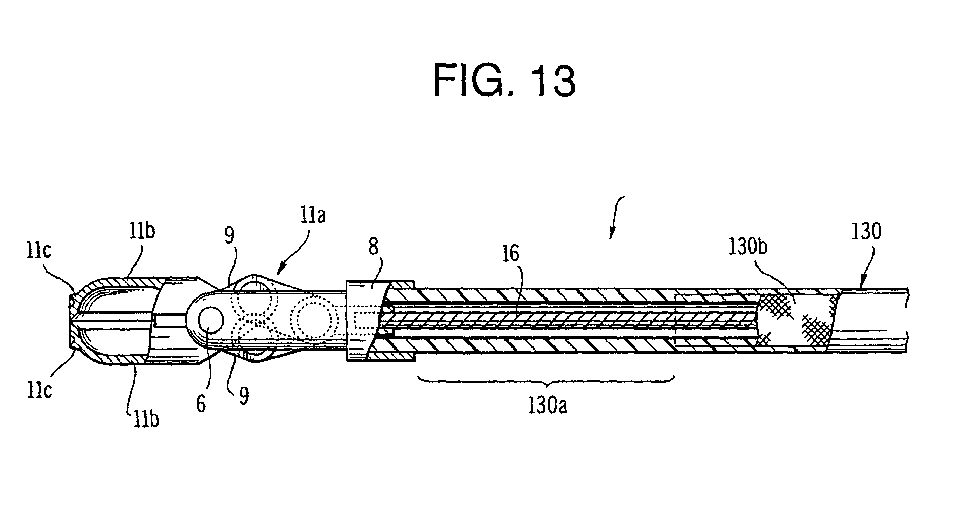

Fig 13 shows a treatment accessory 300 according to a first example which is an embodiment of the invention. The treatment accessory 300 is similar to the treatment accessory 100 of the first example except that the treatment accessory 300 includes a flexible shaft 130 which is a tube made from a Teflon resin such as PTFE, an FEP-fluorocarbon resin, such as PFA; a polyethylene resin such as HDPE; or another synthetic resin, such as nylon. Other elements, including the treating device 11, are the same as the first example.

The shaft 130 is provided with a mesh tube 130b made of mesh metal or meshed stiff plastic wires in order to increase the stiffness of a main portion of the shaft 130. An easy-to-bend (ETB) portion 130a is provided at the distal end portion of the shaft 130 that does not include the mesh tube 130b. As an example, the length of the shaft 130 is 1 - 2 m, and the length of the ETB portion 130a is 5 - 30 mm.

Similar to the first example, the shaft 130 is designed such that the flexibility of the ETB portion 130a is twice that of the main portion of the shaft 130. The operation of the treatment accessory 400 is also similar to that of the treatment accessory 300 of the first example.

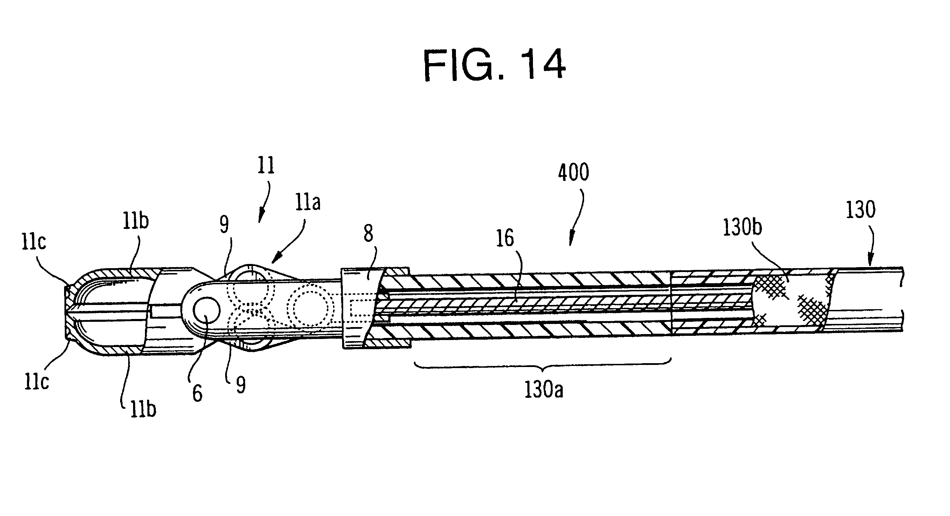

Fig. 14 shows an alternative arrangement (i.e., a fourth example) in which the ETB portion 130a is formed separately from the shaft 130 and the ETB portion 130a and the shaft 13 are then connected by, for example, fusing resin together to obtain a sufficient bonding strength and endurance, or alternatively, using a metal pipe (not shown) or the like, such that the ends of the shaft 13 and the ETB portion 130a are adhered on the outer surface or inner surface of the metal pipe.

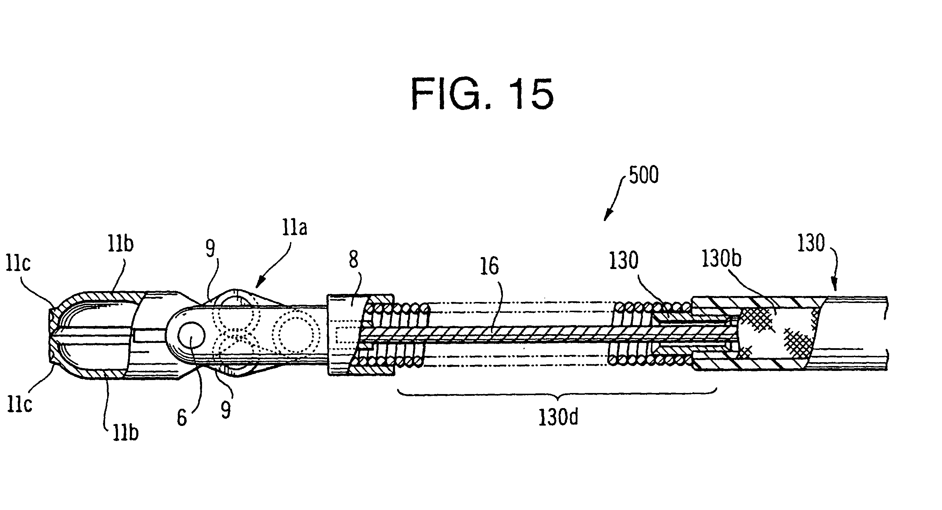

Fig. 15 shows another alternative arrangement (i.e., a fifth example) 500 in which the shaft 130 is made of the synthetic resin and an ETB portion 130d is made of a coiled-wire pipe. As above, the ETB portion 130d uses coiled-wire pipe that is designed to have a relatively small diameter and flexibility that is twice that of the shaft 130. In this arrangement, the shaft 130 and the ETB portion 130d are connected using, for example, a metal pipe 130e provided inside both the shaft 130 and the ETB portion 130d.

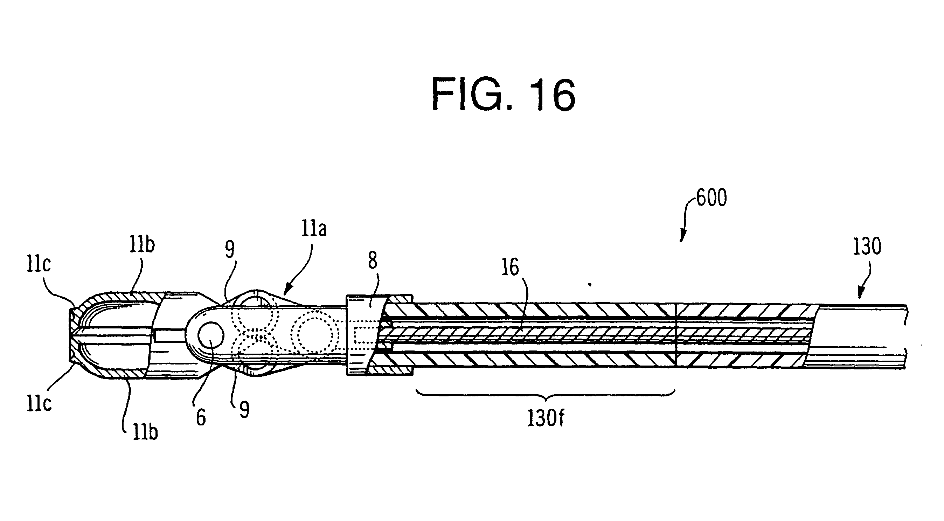

Fig. 16 shows yet another alternative arrangement (i,e., a sixth example) 600 in which the shaft 130 and the ETB portion 130f are formed separately of synthetic resins in order to have different flexibility values. As long as the flexibility condition described above, i.e., that the ETB portion 130f is twice as flexible as the shaft 130, is met, the synthetic resin used may be the same or different. The shaft 130 and the ETB portion 130f are then connected using an appropriate method, for example, using the metal pipe. as shown in Fig. 15. Note that in this arrangement, the mesh tube 130b is not enclosed in the shaft 130.

As a particular example, the ETB portion 130f may be made from soft polyvinyl chloride, polyethylene resin, polyurethane resin, silicon resin or the like, and the shaft 130 may be made from hard polyimide resin, HDPE, nylon resin, Teflon resin or the like.

Of course, as in previous examples, projected portion 11c on the forceps cups 11b may be arranged in a variety of ways. Further, the treating device 11 may be chosen according to the particular treatment required.

According to the above-described examples, and their alternatives, the treatment devices of the treatment accessories can be directed to the affected part, or portion to be treated appropriately with a simple structure. By employing the structure, an operator can easily manipulate both the endoscope and the operation of the treatment accessories.

The extent of protection of the independent claim does not enclose the examples shown in the Figs. 2, 3, 8, 11, 12 and 16. However, these examples are helpful to understand the present invention.

## Claims

1. A treatment apparatus for use with an endoscope, said treatment apparatus being inserted in a forceps channel (2) of said endoscope (1), said treatment apparatus comprising:
a treatment device (11);
an elongated elastic element (13) connected to said treatment device (11) at a distal end of said elastic element (13), said elastic element (13) including at least two bendable portions, a first bendable portion (130b) having a predetermined flexibility, a second bendable portion (130a) having a greater flexibility and being shorter than said first bendable portion (130b), said second bendable portion (130a) being located between said first bendable portion (130b) and said treatment device (11), at least a portion of said first bendable portion (130b) and the entire second bendable portion (130a) protruding from a distal end of said forceps channel (2) when said treatment device (11) is in use, **characterised in that** said first and second bendable portions (130b, 130a) comprise flexible sheaths, and a mesh material being embedded in only said first bendable portion (130b).

2. The treatment apparatus according to claim 1, wherein said second bendable portion (130a) comprises wound coils.

3. The treatment apparatus according to claim 1 or 2 , wherein said first and second bendable portions (130b,130a) are formed of different materials.

4. The treatment apparatus according to claim 1, 2 or 3 , wherein the length of said second bendable (130a) portion is equal to or less than 50mm.

5. The treatment apparatus according to anyone of the foregoing claims, wherein said treatment device (11) has grasping jaws and at least one projection being provided on each of said grasping jaws.

6. The treatment apparatus according to claim 5, wherein said at least one projection does not extend beyond a plane perpendicular to a distal end of said grasping jaws.

7. The treatment apparatus according to claim 5 or 6 , wherein said jaws contact each other at a contact portion when closed, each of said projections being spaced from said contact portion.

8. The treatment apparatus according to anyone of claims 5 through 7, wherein at least one of said projections is half-ring shaped.

9. The treatment apparatus according to anyone of claims 5 through 8, wherein at least one of said projections is cone-shaped.

10. The treatment apparatus according to claims 5 through 9, wherein a plurality of projections are provided on at least one of said jaws.

## Patentansprüche

1. Behandlungseinrichtung zur Verwendung bei einem Endoskop, wobei die Behandlungseinrichtung in einen Zangenkanal (2) des Endoskops (1) eingeführt ist und folgendes umfaßt:
eine Behandlungsvorrichtung (11);
ein längliches elastisches Element (13), das an seinem distalen Ende mit der Behandlungsvorrichtung (11) verbunden ist, wobei das elastische Element (13) zumindest zwei biegbare Abschnitte hat, von denen ein erster biegbarer Abschnitt (130b) eine vorbestimmte Biegsamkeit hat und ein zweiter biegbarer Abschnitt (130a) eine höhere Biegsamkeit hat und kürzer ist als der erste biegbare Abschnitt (130b), wobei der zweite biegbare Abschnitt (130a) zwischen dem ersten biegbaren Abschnitt (130b) und der Behandlungsvorrichtung (11) liegt, und wobei zumindest ein Teil des ersten biegbaren Abschnitts (130b) und der gesamte zweite biegbare Abschnitt (130a) aus einem distalen Ende des Zangenkanals (2) hervorstehen, wenn die Behandlungsvorrichtung (11) in Gebrauch ist, **dadurch gekennzeichnet, daß** der erste und der zweite biegbare Abschnitt (130b, 130a) flexible Hüllen haben und ein Netzmaterial nur in den ersten biegbaren Abschnitt (130b) eingebettet ist.

2. Behandlungseinrichtung nach Anspruch 1, wobei der zweite biegbare Abschnitt (130a) Spiralwicklungen enthält.

3. Behandlungseinrichtung nach Anspruch 1 oder 2, wobei der erste und der zweite biegbare Abschnitt (130b, 130a) aus unterschiedlichen Materialien bestehen.

4. Behandlungseinrichtung nach Anspruch 1, 2 oder 3, wobei die Länge des zweiten biegbaren Abschnitts (130a) gleich oder kleiner als 50 mm ist.

5. Behandlungseinrichtung nach einem der vorhergehenden Ansprüche, wobei die Behandlungsvorrichtung (11) Greifbacken hat und zumindest ein Vorsprung an jeder der Greifbacken vorgesehen ist.

6. Behandlungseinrichtung nach Anspruch 5, wobei der zumindest eine Vorsprung nicht über eine Ebene senkrecht zum distalen Ende der Greifbacken hinaussteht.

7. Behandlungseinrichtung nach Anspruch 5 oder 6, wobei die Backen in geschlossenem Zustand einander in einem Kontaktbereich berühren, wobei jeder Vorsprung von dem Kontaktbereich beabstandet ist.

8. Behandlungseinrichtung nach einem der Ansprüche 5 bis 7, wobei zumindest einer der Vorsprünge halbringförmig ist.

9. Behandlungseinrichtung nach einem der Ansprüche 5 bis 8, wobei zumindest einer der Vorsprünge kegelförmig ist.

10. Behandlungseinrichtung nach den Ansprüchen 5 bis 9, wobei mehrere Vorsprünge an zumindest einer der Backen ausgebildet sind.

## Revendications

1. Appareil de traitement destiné à être utilisé dans un endoscope, ledit appareil de traitement étant inséré dans un canal opérateur (2) dudit endoscope (1), ledit appareil de traitement comportant :
un dispositif de traitement (11) ;
un élément élastique allongé (13) relié audit dispositif de traitement (11) à une extrémité distale dudit élément élastique (13), ledit élément élastique (13) comprenant au moins deux portions pliables, une première portion pliable (130b) présentant une flexibilité prédéterminée, une seconde portion pliable (130a) présentant une plus grande flexibilité et étant plus courte que ladite première portion pliable (130b), ladite seconde portion pliable (130a) étant située entre ladite première portion pliable (130b) et ledit dispositif de traitement (11), au moins une portion de ladite première portion pliable (130b) et toute la seconde portion pliable (130a) faisant saillie d'une extrémité distale dudit canal opérateur (2) lorsque ledit dispositif de traitement (11) est en cours d'utilisation, **caractérisé en ce que** lesdites première et seconde portions pliables (130b, 130a) comportent des gaines flexibles, une matière maillée étant incorporée uniquement dans ladite première portion pliable (130b).

2. Appareil de traitement selon la revendication 1, dans lequel ladite seconde portion pliable (130a) est constituée d'éléments enroulés.

3. Appareil de traitement selon la revendication 1 ou 2, dans lequel lesdites première et seconde portions pliables (130b, 130a) sont formées à partir de matériaux différents.

4. Appareil de traitement selon la revendication 1, 2 ou 3, dans lequel la longueur de ladite seconde portion pliable (130a) est égale ou inférieure à 50 mm.

5. Appareil de traitement selon l'une quelconque des revendications précédentes, dans lequel ledit dispositif de traitement (11) comporte des mâchoires de préhension et au moins une saillie est prévue sur chacune desdites mâchoires de préhension.

6. Appareil de traitement selon la revendication 5, dans lequel ladite saillie au nombre d'au moins un ne s'étend pas au-delà d'un plan perpendiculaire à une extrémité distale desdites mâchoires de préhension.

7. Appareil de traitement selon la revendication 5 ou 6, dans lequel lesdites mâchoires viennent au contact l'une de l'autre au niveau d'une portion de contact lorsqu'elles sont fermées, chacune desdites saillies étant espacée de ladite portion de contact.

8. Appareil de traitement selon l'une quelconque des revendications 5 à 7, dans lequel au moins une desdites saillies a la forme d'un demi-anneau.

9. Appareil de traitement selon l'une quelconque des revendications 5 à 8, dans lequel au moins une desdites saillies a la forme d'un cône.

10. Appareil de traitement selon l'une quelconque des revendications 5 à 9, dans lequel il est prévu une pluralité de saillies sur au moins une desdites mâchoires.
